(19) Europäisches Patentamt
European Patent Office
Office européen des brevets

(11) **EP 4 076 421 B1**

(12) **EUROPEAN PATENT SPECIFICATION**

(45) Date of publication and mention of the grant of the patent:
**26.02.2025 Bulletin 2025/09**

(21) Application number: **20842431.7**

(22) Date of filing: **17.12.2020**

(51) International Patent Classification (IPC):
*A61K 31/167* (2006.01)     *A61K 31/609* (2006.01)
*A61P 33/10* (2006.01)

(52) Cooperative Patent Classification (CPC):
(C-Sets available)
**A61K 31/167; A61K 31/609; A61P 33/10**     (Cont.)

(86) International application number:
**PCT/US2020/065625**

(87) International publication number:
**WO 2021/127189 (24.06.2021 Gazette 2021/25)**

(54) **COMBINATION OF DIAMPHENETHIDE AND CLOSANTEL FOR TREATING LIVER FLUKE INFECTIONS**

KOMBINATION VON DIAMPHENETHIDE UND CLOSANTEL ZUR BEHANDLUNG VON LEBEREGELINFEKTIONEN

COMBINAISON DE DIAMPHENETHIDE ET DE CLOSANTEL POUR LE TRAITEMENT DES INFECTIONS PAR LA DOUVE DU FOIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2019 US 201962949748 P**

(43) Date of publication of application:
**26.10.2022 Bulletin 2022/43**

(73) Proprietor: **Elanco Tiergesundheit AG**
**4058 Basel (CH)**

(72) Inventors:
• **ROLFE, Peter**
**Greenfield, Indiana 46140 (US)**
• **GEORGE, Sarah**
**Greenfield, Indiana 46140 (US)**
• **TAHTAOUI, Chouaib**
**Greenfield, Indiana 46140 (US)**

(74) Representative: **Cohausz & Florack**
**Patent- & Rechtsanwälte**
**Partnerschaftsgesellschaft mbB**
**Bleichstraße 14**
**40211 Düsseldorf (DE)**

(56) References cited:
• **FAIRWEATHER I ET AL: "FASCIOLICIDES: EFFICACY, ACTIONS, RESISTANCE AND ITS MANAGEMENT", VETERINARY JOURNAL, BAILLIERE TINDALL, LONDON, GB, vol. 158, no. 2, 1 September 1999 (1999-09-01), pages 81 - 112, XP008076178, ISSN: 1090-0233, DOI: 10.1053/ TVJL.1999.0377**

**(Cont. next page)**

(52) Cooperative Patent Classification (CPC): (Cont.)

C-Sets
**A61K 31/167, A61K 2300/00;**
**A61K 31/609, A61K 2300/00**

## Description

### BACKGROUND

Liver fluke are helminth parasites that cause a major economic loss in the livestock industry primarily by infection with *Fasciola hepatica* or its tropical counterpart *Fasciola gigantica.* Since all known drugs active against *F. hepatica* are also active against F. *gigantica*, the discussion that follows will refer to them collectively as *Fasciola* spp.

The primary impact of *Fasciola* spp infection is on farmed ruminants, however, they are not selective regarding the final host and successfully infect a wide range of mammals, including humans. Among the farmed ruminants, *Fasciola* spp may cause annual economic losses of several billions of dollars world-wide. McVeigh et al., Trends in Parasitology, March, 2018, Vol. 34, No. 3 184-196.

The liver fluke has a complex life cycle. In its final host *Fasciola* spp can be categorized into three stages, early immature, immature, and mature (commonly termed the adult stage). In sheep the early immature form occurs with flukes of 1-3 weeks of age, the immature form occurs with flukes of 4-7 weeks of age, and the mature form occurs with flukes of 8 weeks and older. In cattle the early immature form occurs with flukes of 1-5 weeks of age, the immature form occurs with flukes of 6-9 weeks of age, and the mature form occurs with flukes of 10 weeks and older.

Some anthelmintic drugs are only effective against the adult parasites often requiring more frequent applications, while many have now lost their advantage in the chemoprophylaxis of fasciolosis due to the development of drug resistance.

Fairweather, Ian, and J. C. Boray. "Fasciolicides: efficacy, actions, resistance and its management." The Veterinary Journal 158.2 (1999): 81-112 teaches modes of action of fasciolicides.

In Australia, drug resistance in *F. hepatica* to several anthelmintic drugs has been demonstrated in the field and laboratory ((1990) Drug resistance in Fasciola hepatica. In: Boray JC, Martin PJ, Roush RT (eds) Resistance of parasites to antiparasitic drugs MSD AGVET, Rahway, N.J., pp 51-60 and D. Boray J.C., De Bono Drug resistance in Fasciola hepatica, Outteridge PM, Richards RB (eds) Australian advances in veterinary science (1989). The Australian Veterinary Association, pp166-169). It has been shown that long and regular use of salicylanilide compounds, particular rafoxanide and closantel in sheep has selected for resistant strains of F. *hepatica* in endemic areas of New South Wales. These strains have been shown to retain their resistant status in cattle and through several passages in sheep. Of seventeen isolates from different geographical regions, ten (58.8 %) showed resistance to rafoxanide at recommended doses in F. *hepatica* and side resistance to closantel was evident. Resistance to drugs was particularly manifested by immature *F.hepatica* but rarely by the adult fluke. Treatments of liver fluke infections with improved efficacy are desired.

### DETAILED DESCRIPTION

The present invention provides for composition for use in a method of treating liver fluke infections in a mammal in need of such treatment comprising administering the combination comprising an effective amount of diamphenethide in combination with an effective amount of closantel wherein the mammal is sheep, and wherein the liver fluke infection is *Fasciola hepatica* or *Fasciola gigantica* Synergistic combinations of diamphenethide and closantel will have advantages regarding the efficacy against susceptible and resistant *Fasciola spp.*

The references to the methods of treatment in this description are to be interpreted as references to compounds, compositions and medicaments of the present invention for use in those methods.

Diamphenethide is also known by the chemical name N-[4-[2-[2-(4-acetamidophenoxy)ethoxy]ethoxy]phenyl] acetamide. The preparation of diamphenethide is well known in the art. For example, US3896235. Diamphenethide has been used against liver flukes and is rapidly metabolized by deacylation to a compound known as DAMD. Diamphenethide has been found to be highly effective against early immature flukes up to 6 weeks of age but shows progressively lower activity against flukes as they develop to maturity. Fairweather et al., The Veterinary Journal 1999, 158, 81-112. Diamphenethide was marketed under the tradename Coriban® for treatment of liver fluke infection in sheep and was removed from the market in the early 1980s. The recommended oral dose for sheep was 80-120 mg/kg.

Closantel is also known by the chemical name N-[5-chloro-4-[(4-chlorophenyl)cyanomethyl]-2-methylphenyl]-2-hydroxy-3,5-diiodobenzamide. The preparation of closantel is well known in the art. Closantel is marketed for use against liver flukes in sheep and cattle, including under the trade name Flukiver®. Closantel in combination with mebendazole is marketed under the tradename Flukiver® Combi for treatment of liver flukes in sheep.

The term "liver fluke infection" refers to infection with *Fasciola hepatica* or *Fasciola gigantica.* Unless otherwise indicated the term includes infection by flukes at any stage of maturity and mixtures of stages of maturity.

The term "mammal" refers to warm-blooded vertebrate animals having hair or fur and secrete milk by the females for the nourishment of the young and includes humans. Particular mammals are ruminants, for example cattle, sheep, goats, bison, African buffalo, water buffalo, antelopes, deer, moose, elks, and giraffes. Also, included in the term mammal are the so-called pseudo-ruminants, for example, llamas and alpacas. Also, included in the term mammal are horses and donkeys. More particularly, mammals are understood to be sheep, goats, bison, African buffalo, water buffalo, and cattle. Even more particularly, mammals are sheep and cattle.

The skilled clinician can readily determine a mammal in need of the present treatment. For example, symptoms include fever, malaise, abdominal pain, eosinophilia, enlarged liver, and abnormal liver tests. Also, infection can be detected by antibody testing, including ELISA testing, in particular of the blood and milk. The infection can also be diagnosed by examining fecal specimens, including by ELISA testing. Moreover, modeling based on season, rainfall, temperature, and other local conditions can be used to predict times when liver fluke infection is more likely.

The term "in combination with" as well as "combination" and "synergistic combination" as used herein are taken to mean that diamphenethide is administered prior to, during, or after the administration of closantel. That is, in the present combination diamphenethide and closantel are administered either sequentially or simultaneously to the mammal. Typically, sequentially administration means administration of either diamphenethide or closantel with three days of administration of the other. More typically, the diamphenethide and closantel are administered within a day of each other and even more typically on the same day. Sequential administration also means administration of either diamphenethide or closantel within an hour, or even minutes, of administration of the other. As a matter of convenience, the diamphenethide and closantel are administered simultaneously.

An effective amount of diamphenethide in combination with an effective amount of closantel is administered to the mammal orally or parenterally. In one embodiment, an effective amount of diamphenethide in combination with an effective amount of closantel is administered orally to the mammal. In one embodiment, an effective amount of diamphenethide in combination with an effective amount of closantel is administered parenterally to the mammal.

Oral administration can include adjuvants conventionally used in the art of formulation and may therefore be processed in a known manner to give, for example, solutions, emulsions, soluble powders, powder mixtures, granules or microencapsulation in polymeric substances. Such formulations, preparations or compositions containing diamphenethide and closantel, either separately or together, optionally include a solid or liquid adjuvant, and are produced in a manner well-known in the art, for example by intimately mixing and/or grinding the active ingredients with the adjuvants, for example with solvents, solid carriers, etc. Oral administration can also be accomplished by drench, gavage, tablet, capsule, or in feed.

The terms "effective amount of diamphenethide" and "effective amount of closantel" are taken to mean the amounts of diamphenethide and closantel necessary to either eliminate, nearly eliminate, slow, or arrest the progression of liver fluke infection when administered in the present combination.

In certain embodiments, the effective amount of diamphenethide in the present combination is 20-120 mg/kg (e.g., 20 mg/kg, 25 mg/kg, 30 mg/kg, 35 mg/kg, 40 mg/kg, 45 mg/kg, 50 mg/kg, 55 mg/kg, 60 mg/kg, 65 mg/kg, 70 mg/kg, 75 mg/kg, 80 mg/kg, 85 mg/kg, 90 mg/kg, 95 mg/kg, 100 mg/kg, 105 mg/kg, 110 mg/kg, 115 mg/kg, or 120 mg/kg), and the effective amount of closantel in the present combination is 1-10 mg/kg (e.g, 1 mg/kg, 1.5 mg/kg, 2 mg/kg, 2.5 mg/kg, 3 mg/kg, 3.5 mg/kg, 4 mg/kg, 4.5 mg/kg, 5 mg/kg, 5.5 mg/kg, 6 mg/kg, 6.5 mg/kg, 7 mg/kg, 7.5 mg/kg, 8 mg/kg, 8.5 mg/kg, 9 mg/kg, 9.5 mg/kg, or 10 mg/kg). In certain embodiments, the effective amount of diamphenethide in the present combination is 20-80 mg/kg (e.g., 20 mg/kg, 30 mg/kg, 40 mg/kg, 50 mg/kg, 60 mg/kg, 70 mg/kg, or 80 mg/kg), and the effective amount of closantel in the present combination is 1-10 mg/kg (e.g, 1.5 mg/kg, 3.5 mg/kg, 5 mg/kg, 7 mg/kg, or 10 mg/kg). In certain embodiments, the effective amount of diamphenethide in the present combination is 30-80 mg/kg, 40-80 mg/kg, 50-80 mg/kg, 60-80 mg/kg, or 70-80 mg/kg. In certain embodiments, the effective amount of closantel in the present combination is 1.5-10 mg/kg, 3-10 mg/kg, 5-10 mg/kg, or 7-10 mg/kg. In certain embodiments, the effective amount of diamphenethide in the present combination is 30-60 mg/kg, 40-60 mg/kg, or 40-50 mg/kg. In certain embodiments, the effective amount of closantel in the present combination is 3-10 mg/kg, 5-10 mg/kg, 6-10 mg/kg, or 7-10 mg/kg.

In certain embodiments, for treatment of sheep the effective amount of diamphenethide in the present combination is 20-80 mg/kg and the effective amount of closantel in the present combination is 3.5-10 mg/kg. In certain embodiments, for treatment of sheep the effective amount of diamphenethide in the present combination is 20-60 mg/kg and the effective amount of closantel in the present combination is 5-10 mg/kg. In certain embodiments, for treatment of sheep the effective amount of diamphenethide in the present combination is 35 mg/kg and the effective amount of closantel in the present combination is 5 mg/kg.

Also described herein, but not encompassed by the claims, is the treatment of cattle wherein the effective amount of diamphenethide in the present combination is 40-120 mg/kg and the effective amount of closantel in the present combination is 3.5-10 mg/kg. Also described herein, but not encompassed by the claims, is the treatment of cattle wherein the effective amount of diamphenethide in the present combination is 40-80 mg/kg and the effective amount of closantel in the present combination is 5-10 mg/kg. Also described herein, but not encompassed by the claims, is the treatment of cattle wherein the effective amount of diamphenethide in the present combination is 70 mg/kg and the effective amount of closantel in the present combination is 10 mg/kg.

The present invention provides for composition for use in a method of treating liver fluke infections in a mammal in need of such treatment comprising administering the composition comprising an effective amount of diamphenethide in combination with an effective amount of closantel

wherein the mammal is sheep, and wherein the liver fluke infection is *Fasciola hepatica* or *Fasciola gigantica.* The present invention pertains to (although not all is part of the claimed invention which is defined in the claims) mammals, wherein the mammal is a ruminant. The treated mammal according to the invention is sheep. Also described herein, but not encompassed by the claims is that the mammal is cattle. In another embodiment, the effective amount of diamphenethide in the combination is 20-80 mg/kg (e.g., 20 mg/kg, 30 mg/kg, 40 mg/kg, 50 mg/kg, 60 mg/kg, 70 mg/kg, or 80 mg/kg). In another embodiment, the effective dose of closantel in the combination is 1-10 mg/kg (e.g, 1.5 mg/kg, 3.5 mg/kg, 5 mg/kg, 7 mg/kg, or 10 mg/kg). In certain embodiments, the effective amount of diamphenethide is 30-80 mg/kg, 40-80 mg/kg, 50-80 mg/kg, 60-80 mg/kg, or 70-80 mg/kg. In certain embodiments, the effective amount of closantel is 1.5-10 mg/kg, 3-10 mg/kg, 5-10 mg/kg, or 7-10 mg/kg.

Also described herein is the use of a combination of diamphenethide and closantel in the manufacture of a medicament for the treatment of liver fluke infections. In another embodiment, the use of diamphenethide in the combination is 20-80 mg/kg (e.g., 20 mg/kg, 30 mg/kg, 40 mg/kg, 50 mg/kg, 60 mg/kg, 70 mg/kg, or 80 mg/kg). In another embodiment, the use of closantel in the combination is 1-10 mg/kg (e.g, 1.5 mg/kg, 3.5 mg/kg, 5 mg/kg, 7 mg/kg, or 10 mg/kg). In certain embodiments, the effective amount of diamphenethide is 30-80 mg/kg, 40-80 mg/kg, 50-80 mg/kg, 60-80 mg/kg, or 70-80 mg/kg. In certain embodiments, the effective amount of closantel is 1.5-10 mg/kg, 3-10 mg/kg, 5-10 mg/kg, or 7-10 mg/kg.

The following examples illustrate the improved efficacy of the combination of diamphenethide and closantel in sheep and cattle. It is understood that the examples are set forth by way of illustration and not limitation.

In the following examples, all animals were confirmed free of infection with F. *hepatica* and found clinically healthy by a veterinarian prior to enrollment in the study. Doses were administered orally from suitably sized disposable syringes.

The fluke counts and body weights were log-transformed (after adding a constant of 1 to the fluke counts only) and the distribution of the transformed data checked to see if they satisfied the assumption of normal distribution. Differences between treatment groups were analyzed by analysis of variance methods (ANOVA), as the assumptions of normal distribution were satisfied for log-transformed fluke count and log-transformed bodyweight, as well as for (untransformed) fluke measurements.

Arithmetic means (Amean), geometric means (Gmean), median and ranges were summarized for fluke counts, fluke measurements and bodyweight. A constant of one was applied for calculation of geometric means of fluke counts.

The percentage efficacy for each treated group was calculated as follows:

$$\% \text{ Efficacy} = 100 \text{ x } (FC_C - FC_T) / FC_C$$

where $FC_C$ is the arithmetic mean fluke count of the control group and $FC_T$ is the arithmetic mean fluke count of the treated group. The formula was also applied using mean geometric fluke counts; these were calculated using log transformed data with 1 added as a constant to each fluke count and then subtracted following transformation.

A synergistic effect was concluded if:

$$(1 - \text{Efficacy}_{D+C}) < (1 - \text{Efficacy}_D) \text{ x } (1 - \text{Efficacy}_C)$$

where D=Diamphenethide and C=Closantel and efficacies are measured as absolute numbers (not in %).

Note that expected synergy is defined as:

$$1 - \text{Efficacy}(\text{expected without synergy}) = (1 - \text{Efficacy}_D) \text{ x } (1 - \text{Efficacy}_C).$$

Example 1

Typical formulations of diamphenethide and closantel are provided in Example 1. Suspension of diamphenethide was prepared as follows: place propylene glycol into a glass beaker. Add both parabenes and stir until dissolved. Add Polysorbate 80, antifoam emulsion and water and stir until homogeneity is reached. Add Avicel RC591 very slowly while stirring. Afterward homogenize with an Ultra-Turrax® for several minutes, add diamphenethide slowly while stirring and homogenize with an Ultra-Turrax® again for at least 10 minutes to give a suspension having the composition below:

1. Diamphenethide 15% (w/v)
2. Propylene glycol 5% (w/v)
3. Methylparaben 0.11% (w/v)
4. Propylparaben 0.04% (w/v)

5. Polysorbate 80 0.5% (w/v)
6. Avicel RC 591 1.2% (w/v)
7. Antifoam emulsion 0.6% (w/v)
8. Demineralised water ad 100.

Suspension of closantel can be prepared as follows: Heat water, add both parabenes and stir until they are dissolved. Cool to ambient temperature and add SDS and antifoam emulsion and stir until homogeneity is reached. Add Avicel RC591 very slowly while stirring. Afterward homogenize with an Ultra-Turrax® for several minutes Add closantel slowly while stirring and homogenize with an Ultra-Turrax® again for at least 10 minutes to give a suspension having the composition below:

1. Closantel 3.75% (w/v)
2. Methylparaben 0.11% (w/v)
3. Propylparaben 0.04% (w/v)
4. SDS (sodium lauryl sulfate) 0.1% (w/v)
5. Avicel RC 591 1.2% (w/v)
6. Antifoam emulsion 0.5% (w/v)
7. Demineralised water ad 100.

Example 2

Efficacy of Diamphenethide in Combination with Closantel in Oral Formulations Against the Early Immature Stage of *Fasciola hepatica* in Sheep

This study was to assess the efficacy of diamphenethide (60 mg/kg) given concurrently with closantel (10 mg/kg), diamphenethide alone (60 mg/kg), and closantel alone (10 mg/kg) against F. *hepatica* in sheep with treatment on day 14. This was a controlled, blinded, randomized study using 35-6 month old Merino lambs.

On day 0, the sheep were experimentally infected with a target of 87 Oberon isolate (Fashep-65) *F. hepatica* metacercariae. The required number of metacercariae were prepared for infection in a water and 4% carboxymethylcellulose solution. Each infective dose was followed by water administered orally.

The sheep were randomized into treatment groups on the basis of day 11 body weight and allocated to groups as denoted in Table 2.1.

Table 2.1

| Group | Treatment | Dose (mg/kg) | n |
|-------|-----------|--------------|---|
| 1 | Untreated | - | 3 |
| 2 | Diamphenethide | 60 | 3 |
| 3 | Closantel | 10 | 3 |
| 4 | Diamphenethide and Closantel | 60 and 10 | 3 |

Body weight was determined on day 11 and doses were measured by volume and rounded to the nearest 0.1 mL. On day 14, and before feeding, the individual animals were treated once only with the Treatment in Table 2.1. Once the animals had been treated they were returned to their pens and fed within one hour. Untreated control animals were also fed at this time.

The liver with gall bladder and common bile duct intact were recovered on day 91 or 92 from each animal and processed for *F. hepatica* counts. The assessment of efficacy was based on the number of live fluke removed from the liver.

An ANOVA model was used to compare fluke counts between treatment groups. The model was applied to the log-transformed counts. There was one fixed effect, treatment group.

All control sheep in Group 1 were positive for Oberon *F. hepatica* infection, with an Arithmetic mean of 20 and Geometric mean of 16. The percentage efficacy for treated sheep were compared to those of the sheep in the untreated control group as shown in Table 2.2 below.

EP 4 076 421 B1

Table 2.2

| Group | Treatment | Fluke Count[1] | % Efficacy[2] | Fluke Count[3] | % Efficacy[4] |
|---|---|---|---|---|---|
| 1 | Untreated Control | 19.7 | - | 16.3 | - |
| 2 | Diamphenethide | 0.3 | 98.3 | 0.3 | 98.4 |
| 3 | Closantel | 15.7 | 20.3 | 14.5 | 10.6 |
| 4 | Diamphenethide and Closantel | 0 | 100 | 0 | 100 |
| [1] Arithmetic mean; [2] Using Arithmetric Mean; [3] Geometric mean; [4] Using Geometric Mean | | | | | |

Example 3

Evaluation of the Synergistic Effect of Diamphenethide Administered Concurrently with Closantel Against the Immature Stage of *Fasciola hepatica* Infections in Sheep

This study was to assess the efficacy of diamphenethide (40 mg/kg) given concurrently with closantel (3.75 mg/kg), diamphenethide alone (40 mg/kg), and closantel alone (3.75 mg/kg) against *F. hepatica* in sheep (3-4 month Merino lambs) with treatment on day 42. This was a controlled, blinded, randomized study.

On day 0, the sheep were experimentally infected with a single isolate of approximately 200 Sunny Corner (Fashep-66) isolate *F. hepatica* metacercariae. The required number of metacerceriae were made up in carboxymethyl cellulose. Syringes were checked post-infection for retained metacerceriae. The infection was administered orally and each infective dose was followed by water administered orally. The sheep were randomized into treatment groups on the basis of day 41 body weight and allocated to groups as denoted in Table 3.1.

Table 3.1

| Group | Treatment | Dose (mg/kg) | n |
|---|---|---|---|
| 1 | Untreated | - | 5 |
| 2 | Diamphenethide | 40 | 5 |
| 3 | Closantel | 3.75 | 5 |
| 4 | Diamphenethide and Closantel | 40 and 3.75 | 5 |

Body weight was determined one day prior to treatment. All treatments were measured by volume and rounded to the nearest 0.1 mL. On day 42, and before feeding, the individual animals were treated once only with the treatment in Table 3.1. Once the animals had been treated they were returned to their pens and fed within one hour. Untreated control animals were also fed at this time.

The liver with gall bladder and bile ducts intact were recovered from each animal on day 91 and processed for *F. hepatica* counts. The assessment of efficacy was based on the number of live fluke removed from the liver.

All control animals were positive for *F. hepatica* infection, with an Arithmetic mean of 90.2 and Geometric mean of 87.02. The percentage efficacy for treated sheep were compared to those of the sheep in the untreated control group as shown in Table 3.2 below.

Table 3.2

| Group | Treatment | Fluke Count[1] | % Efficacy[2] | Fluke Count[3] | % Efficacy[4] |
|---|---|---|---|---|---|
| 1 | Untreated Control | 90.2 | - | 87.02 | - |
| 2 | Diamphenethide | 57.6 | 36.1 | 50.9 | 41.5 |
| 3 | Closantel | 54.0 | 40.1 | 51.6 | 40.7 |
| 4 | Diamphenethide and Closantel | 23.4 | 74.1 | 20.9 | 76.0 |
| [1] Arithmetic mean; [2] Using Arithmetic mean; [3] Geometric Mean; [4] Using Geometric Mean | | | | | |

Pair-wise comparisons demonstrated the concurrent treatment to have significantly lower fluke counts than the untreated and single formulation treated groups and the single active treatments were not significantly different from each other, in regards to fluke count, as shown in Table 3.3.

Table 3.3

| Group | 2 | 3 | 4 |
|---|---|---|---|
| 1 | 0.1947 | 0.2051 | 0.0017 |
| 2 | | 0.9751 | 0.0387 |
| 3 | | | 0.0362 |

Expected and observed % Efficacy against *F. hepatica* for concurrent treatments is presented in Table 3.4.

Table 3.4

| Group | Treatment | Amean | | Gmean | |
|---|---|---|---|---|---|
| | | Expected | Observed | Expected | Observed |
| 4 | Diamphenethide + Closantel | 61.8 | 74.1 | 65.3 | 76.0 |

The observed efficacy of concurrent treatment of diamphenethide and closantel was higher than the expected additive effect demonstrating a synergistic effect.

**Claims**

1. A composition for use in a method of treating liver fluke infections in a mammal in need of such treatment comprising administering the composition comprising an effective amount of diamphenethide in combination with an effective amount of closantel

   wherein the mammal is sheep, and
   wherein the liver fluke infection is *Fasciola hepatica* or *Fasciola gigantica.*

2. The composition for use according to claim 1, wherein the effective amount of diamphenethide is 20-80 mg/kg.

3. The composition for use according to claim 1 or 2, wherein the effective amount of diamphenethide is 20 mg/kg, 30 mg/kg, 40 mg/kg, 50 mg/kg, 60 mg/kg, 70 mg/kg, or 80 mg/kg.

4. The composition for use according to any one of claims 1-3, wherein the effective amount of closantel is 3-10 mg/kg.

5. The composition for use according to any one of claims 1-4, wherein the effective amount of closantel is 3.5 mg/kg, 5 mg/kg, 7 mg/kg, or 10 mg/kg.

**Patentansprüche**

1. Zusammensetzung zur Verwendung in einem Verfahren zur Behandlung von Leberegelinfektionen bei einem Säugetier, das einer solchen Behandlung bedarf, umfassend die Verabreichung der Zusammensetzung, die eine wirksame Menge von Diamphenethid in Kombination mit einer wirksamen Menge von Closantel umfasst, wobei das Säugetier ein Schaf ist und
   wobei die Leberegelinfektion *Fasciola hepatica* oder *Fasciola gigantica* ist.

2. Die Zusammensetzung zur Verwendung nach Anspruch 1, wobei die wirksame Menge an Diamphenethid 20-80 mg/kg beträgt.

3. Die Zusammensetzung zur Verwendung nach Anspruch 1 oder 2, wobei die wirksame Menge an Diamphenethid 20 mg/kg, 30 mg/kg, 40 mg/kg, 50 mg/kg, 60 mg/kg, 70 mg/kg oder 80 mg/kg beträgt.

4. Die Zusammensetzung zur Verwendung nach einem der Ansprüche 1 bis 3, wobei die wirksame Menge an Closantel 3 bis 10 mg/kg beträgt.

**5.** Die Zusammensetzung zur Verwendung nach einem der Ansprüche 1-4, wobei die wirksame Menge an Closantel 3,5 mg/kg, 5 mg/kg, 7 mg/kg oder 10 mg/kg beträgt.

**Revendications**

**1.** Composition destinée à être utilisée dans une méthode de traitement des infections de la douve du foie chez un mammifère nécessitant un tel traitement, comprenant l'administration de la composition comprenant une quantité efficace de diamphénéthide en combinaison avec une quantité efficace de closantel, le mammifère étant un mouton, et l'infection de la douve du foie étant *Fasciola hepatica* ou *Fasciola gigantica.*

**2.** La composition à utiliser selon la revendication 1, dans laquelle la quantité efficace de diamphénéthide est de 20 à 80 mg/kg.

**3.** La composition à utiliser selon la revendication 1 ou 2, dans laquelle la quantité efficace de diamphénéthide est de 20 mg/kg, 30 mg/kg, 40 mg/kg, 50 mg/kg, 60 mg/kg, 70 mg/kg ou 80 mg/kg.

**4.** La composition à utiliser selon l'une des revendications 1 à 3, dans laquelle la quantité efficace de closantel est de 3 à 10 mg/kg.

**5.** La composition à utiliser selon l'une des revendications 1 à 4, dans laquelle la quantité efficace de closantel est de 3,5 mg/kg, 5 mg/kg, 7 mg/kg ou 10 mg/kg.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3896235 A

**Non-patent literature cited in the description**

- **MCVEIGH et al.** *Trends in Parasitology*, 2018, vol. 34 (3), 184-196
- **FAIRWEATHER, IAN** ; **J. C. BORAY**. Fasciolicides: efficacy, actions, resistance and its management.. *The Veterinary Journal*, 1999, vol. 158 (2), 81-112
- Drug resistance in Fasciola hepatica.. Resistance of parasites to antiparasitic drugs MSD AGVET. 1990, 51-60
- De Bono Drug resistance in Fasciola hepatica. **D. BORAY J.C.** Australian advances in veterinary science. The Australian Veterinary Association, 1989, 166-169
- **FAIRWEATHER et al.** *The Veterinary Journal*, 1999, vol. 158, 81-112